Europäisches Patentamt

European Patent Office

Office européen des brevets

(19)

(11) Publication number: **0 060 719**
**B1**

(12) **EUROPEAN PATENT SPECIFICATION**

(45) Date of publication of patent specification: **27.11.85**

(51) Int. Cl.⁴: **C 07 C 69/14, C 07 C 67/08**

(21) Application number: **82301333.9**

(22) Date of filing: **16.03.82**

(54) **Process for the production of methyl acetate by esterifying methanol with acetic acid.**

(30) Priority: **17.03.81 GB 8108336**

(43) Date of publication of application:
**22.09.82 Bulletin 82/38**

(45) Publication of the grant of the patent:
**27.11.85 Bulletin 85/48**

(84) Designated Contracting States:
**BE DE FR GB IT NL**

(56) References cited:
**FR-A-1 194 137**
**US-A-2 787 636**

(73) Proprietor: **BP Chemicals Limited**
**Belgrave House 76 Buckingham Palace Road**
**London, SW1W 0SU (GB)**

(72) Inventor: **Yeomans, Bertram**
**BP Chemicals Limited Saltend**
**Hedon Hull, HU12 8DS (GB)**

(74) Representative: **Harry, John et al**
**c/o The British Petroleum Company plc Patents**
**Division Chertsey Road**
**Sunbury-on-Thames Middlesex TW16 7LN (GB)**

Courier Press, Leamington Spa, England.

## Description

The present invention relates to an improved process for the production of methyl acetate by esterifying methanol with acetic acid.

Methyl acetate, besides retaining its usefulness in solvent applications, is gaining importance as an intermediate in the production of other valuable products. Owing to its unfavourable physical properties (viz. composition of azeotropes and solubility in water) pure methyl acetate containing low concentrations of methanol and water is not readily obtainable using conventional processes. A conventional esterification process which may be used for the production of methyl acetate comprises the addition of a feed mixture comprising excess methanol and acetic acid to an esterifier containing aqueous acetic acid and a strong acid catalyst (e.g. toluene-para-sulphonic acid) under reflux conditions. A mixture of methyl acetate, reaction water and the excess methanol is taken overhead as a distillate product. A typical ester distillate product obtained by use of a 2:1 molar ratio feed of methanol:acetic acid should theoretically comprise 59.9% w/w methyl acetate, 26% w/w methanol and 14.5% w/w water. Conventionally purification of such a crude ester distillate is effected in a multi-stage process which typically incorporates hydroselection (for removal of methanol) and azeotropic drying (for removal of water). Additional distillation steps are required to recover methanol from the hydroselection base product and methyl acetate from the decanter water phase/entrainer for recycle.

US 2,787,636 describes the addition of an auxiliary liquid, for example butyl acetate, to an esterification process. However, in this case the auxiliary liquid allows the esterification reaction to be carried out at an elevated temperature. The auxiliary liquid also remains in the esterification reactor throughout the reaction and does not act as an entrainer.

French Patent 1,194,137 also described an esterification process using, for example, butyl acetate as an entrainer but in this patent the entrainer is removed overhead from the distillation column and not at an intermediate point.

We have now found that methyl acetate of improved purity in terms of methanol and water content and hence of greater suitability as a feedstock in certain reactions, such as the production of acetic anhydride by reaction with carbon monoxide, can be produced in a simpler manner than hitherto.

Accordingly, the present invention provides a process for the production of methyl acetate which process comprises reacting in an esterification reaction vessel methanol at elevated temperature with acetic acid in the presence of an esterification catalyst and an entrainer which is sparingly soluble in water and which forms a minimum boiling point azeotrope therewith to form a product comprising entrainer, methyl acetate and water, and in a distillation column, recovering from the product an overhead fraction comprising methyl acetate characterised in that from an intermediate point in the column there is removed a liquid sidestream fraction comprising water and entrainer.

The entrainer may suitably be added to the esterification reaction vessel or to a suitable point in the distillation column. The process may be operated batchwise or continuously preferably continuously.

A preferred embodiment is characterised in that the methanol and acetic acid are continuously fed to the esterification reaction vessel containing entrainer, acetic acid and esterification catalyst to produce a product comprising entrainer, unreacted methanol, methyl acetate and water, distilling from the product in the distillation column an overhead fraction comprising methyl acetate, removing from the intermediate point in the column a liquid sidestream fraction comprising water, entrainer and methyl acetate, separating methyl acetate and entrainer in the sidestream fraction from water and returning separated methyl acetate and entrainer to a point lower in the column than the sidestream fraction removal point.

The esterification reaction vessel may be separate from the distillation column or integral therewith. Preferably the vessel is a kettle at the base of the distillation column, which suitably may contain not less than 8 theoretical plates and preferably from 15 to 50 theoretical plates. Whenever, the reaction vessel is separate from the distillation column it is preferred to recycle the residue from the base of the column to the reaction vessel.

Preferably the feed comprises equimolar amounts of methanol and acetic acid.

The elevated temperature may vary over a moderately wide range but must be sufficient, in the case where the reaction vessel is integral with the distillation column, to distil methyl acetate, water and entrainer out of the reaction mixture. Thus at atmospheric pressure suitable reaction temperatures are in the range 100° to 120°C, preferably 105° to 115°C. To achieve the elevated temperature the reaction vessel may be provided with, for example, steam coils, or other forms of heating.

The reflux necessary for successful fractionation may suitably be provided by condensing the overhead fraction in a vertical condenser and returning a portion of the condensate to the column (primary reflux), the remainder of the condensate being removed as methyl acetate product. The primary reflux ratio may suitably be in the range 1:2 to 10:1, preferably from 1:1 to 5:1.

In the embodiment in which the reaction vessel constitutes the kettle of the distillation column the reflux ratio is preferably sufficient to maintain the water content of the reaction vessel at not more than 10% by weight of the reactor contents and preferably not more than 3% by weight.

Operating at atmospheric pressure the side- stream fraction is preferably removed at a point in the column at which the column temperature is between 75 and 90°C. In terms of theoretical plates this point should be not less than 5 and preferably not less than 10 theoretical plates from the base of the column. In addition to water and entrainer, the liquid sidestream fraction may additionally comprise methyl acetate. This may be separated, suitably by decantation, into an upper organic phase comprising methyl acetate and entrainer which may be recycled back to the column and a lower aqueous phase comprising water. The aqueous phase may suitably be discarded.

The distillation column may suitably incorporate means for facilitating the removal of a liquid sidestream fraction which means may take the form of a deep weir or chimney tray located immediately below the point in the column from which the sidestream fraction is removed. Using such means it may be possible to maximise the concentration of water in the sidestream fraction by facilitating phase separation within the column, thereby saving energy.

Since substantially all the entrainer remains within the column, only small amounts being lost in the overhead fraction and the sidestream fraction aqueous phase, it is preferred to feed entrainer in small amounts to the esterification reaction vessel to replace that lost from the column.

A particularly preferred embodiment of the present invention comprises continuously feeding methanol and acetic acid to a reaction kettle surmounted by a distillation column, which kettle contains entrainer, acetic acid and esterification catalyst at elevated temperature, thereby producing a product comprising methyl acetate, water, unreacted methanol, unreacted acetic acid, catalyst and entrainer, removing from the column an overhead fraction comprising principally methyl acetate together with some methanol and water, condensing the overhead fraction, characterised in that the process comprises returning a portion of the condensed overhead fraction to the column as primary reflux leaving in the reaction kettle a residue fraction comprising catalyst, acetic acid, entrainer and some water, removing from an intermediate point in the liquid sidestream fraction comprising water, methyl acetate and entrainer together with some methanol, separating the sidestream fraction by decantation into an upper organic phase comprising principally entrainer and some methyl acetate and a lower aqueous phase comprising principally water and some methyl acetate and methanol and returning the separated organic phase to the column at a point lower in the column than the sidestream fraction removal point.

The methyl acetate recovered overhead may contain impurities of which methanol and water form the major proportion. The methyl acetate may be further purified if so desired by hydroselection (for removal of methanol) and/or azeotropic drying (for removal of water) and/or selective adsorption using a molecular sieve and/or by reaction with acetic anhydride.

With respect to the reactants, it is immaterial whether the methanol and/or acetic acid contain water.

The entrainer may be any hydrocarbon, ether, ester or ketone which is sparingly soluble in water and which forms a minimum boiling point azeotrope with water. Examples of suitable entrainers are toluene, diisobutyl ether, normal- and isobutyl acetate and methyl isobutyl ketone. Preferably the entrainer is an acetic acid ester such as n-butyl acetate. An advantage of using an acetic acid ester is that it can be formed 'in situ', for example n-butyl acetate can be formed by incorporating n-butanol in the reaction mixture. Methyl acetate (boiling point 57°C) and butyl acetate (boiling point 126°C) can be readily separated by distillation.

The amount of entrainer present may suitably be greater than 1%, preferably from 20 to 60% by weight based on the total weight of the reaction mixture.

The esterification catalyst may suitably be a mineral acid such as sulphuric acid or an organic acid such as toluene-para-sulphonic acid, of which toluene-para-sulphonic acid is preferred. The esterification catalyst may be present in an amount from 0.1 to 10%, preferably from 2 to 6% by weight of the reaction mixture.

The aforegoing description relates only to atmospheric pressure operation. The corresponding operating conditions required for operation under any reduced or superatmospheric conditions may be readily determined by extrapolation of the data, or by experiment using the preferred atmospheric conditions as a guideline.

The recovered methyl acetate is suitable, with or without further purification, as feed to a process for producing acetic anhydride and/or acetic acid by reaction with carbon monoxide in the presence of a metallic carbonylation catalyst, eg a Group VIII noble metal, of which rhodium is preferred, and a promoter comprising a halogen in free or combined formed, eg iodine or methyl iodide or acetyl iodide, and optionally in the presence also of co-promoters, such as alkyl or aryl phosphines and organo-nitrogen compounds. Suitable catalysts, promoters, co-promoters and reaction conditions are described in for example UK Patents Nos. 1468940, 1523346 and 1538783 and European Patent No. 8396.

The invention will now be further illustrated by reference to the following Example.

Example 1

A feed mixture (6000 g) comprising 63.35% w/w acetic acid, 35.65% w/w methanol and 1% w/w n-butyl acetate was fed over 9.5 hours into a reaction still kettle (containing 800 g of refluxing mixture comprising 64.9% w/w acetic acid 29.35% w/w n-butyl acetate, 1.0% w/w water and 4.75% w/w toluene-para-sulphonic acid), fitted with a 25-plate Oldershaw column and overhead product condensation/reflux control system. The reaction still was operated to give a reflux temperature within the range 80 to 90°C at plate 15. The column also contained a deep weir sited immediately above plate 15 from the kettle. A liquid sidestream was removed from the deep weir, cooled and fed to a decantation vessel wherein separation into a water phase and an oil phase occurred. The oil phase was recycled back to the column on to plate 15. The separated water phase (1300 g) consisting of methyl acetate (13.5% w/w), n-butyl acetate (0.9% w/w), methanol (1.7 w/w), acetic acid (trace) and water (83.9% w/w) was discharged. The methyl acetate product (4700 g) was taken overhead using a reflux ratio of ca. 8:1 and this comprised 95.7% w/w methyl acetate, 1.7% w/w aethanol, less than 0.05% w/w n-butyl acetate, 0.002% w/w acetic acid and 2.6% w/w water. The small concentration of n-butyl acetate used in the feed was required to balance that leaving the reaction still dissolved in the aqueous side-stream and thus maintain the high concentration present around the weir which was required to give an efficient separation of water phase.

Comparison test

A feed mixture (4122 g) comprising 48.4% w/w acetic acid and 51.6% w/w methanol (equivalent to a molar ratio of 1:2 respectively) was fed over 6.5 hours into an esterifier (ca. 2 l capacity) containing a refluxing mixture (800 g) of 50% w/w acetic acid, 48% w/w water and 2% w/w toluene-para-sulphonic acid and fitted with a 5-plate Oldershaw column. The methyl acetate product (4123 g), which comprised 57.5% w/w methyl acetate, 27.9% w/w methanol, 13.8% w/w water and 0.8% w/w acetic acid, was taken overhead using a reflux ratio of 3:1 and a stillhead temperature of ca. 70°C/l bar.

This is not an example according to the invention because no entrainer was employed and no sidestream was removed from the column. The Example demonstrates that by operating according to the present invention methyl acetate of purity 95.7% w/w can be obtained in a single column whereas using the conventional process the methyl acetate recovered was only 57.5% w/w pure.

Example 2

Using the reaction still described in Example 1, the kettle was charged with acetic acid 56.7% w/w, n-butyl acetate 22.5% w/w, methyl acetate 10.4% w/w, toluene-p-sulphonic acid 6.3% w/w, and water 4.2% w/w, and n-butyl acetate 1.3% w/w was fed continuously into the reaction still, which was operated to give a temperature of 79°C at plate 15. The liquid sidestream removed from the deep weir was cooled and fed to the decantation vessel wherein separation into two phases occurred. The oil phase was recycled back to the column on to plate 15. The separated water phase (1050 g) consisting of methyl acetate (13.0% w/w, n-butyl acetate (0.5% w/w), methanol (4.3% w/w), acetic acid (0.8% w/w) and water (81.3% w/w) was discharged. The methyl acetate product (3720 g) was taken overhead using a primary reflux ratio of ca. 5:1 and this comprised 94.5% w/w methyl acetate, 3.4% w/w methanol, 0.02% w/w n-butyl acetate, and 2.0% w/w water.

Example 3

The reaction still was charged as in Example 2 and the contents brought to reflux. Over a period of 16 hours, 8050 g of a feed comprising acetic acid 60.7% w/w, methanol 37.8% w/w and n-butyl acetate 1.5% w/w was fed continuously to the reaction still, which was operated to give a temperature of 84°C at plate 15. The liquid sidestream removed from the deep weir was cooled and fed to the decantation vessel wherein separation into two phases occurred. The oil phase was recycled back to the column on to plate 15. The separated water phase (1800 g) consisting of methyl acetate (12.3% w/w), n-butyl acetate (0.6% w/w), methanol (3.8% w/w), acetic acid (0.5% w/w) and water (83.0% w/w) was discharged. The methyl acetate product (6880 g) was taken overhead using a reflux ratio of ca. 2.5:1 and this comprised 94.6% w/w methyl acetate, 2.3% w/w methanol, 0.15% w/w n-butyl acetate, 0.05% w/w acetic acid and 2.9% w/w water.

**Claims**

1. A process for the production of methyl acetate which process comprises reacting in an esterification reaction vessel methanol at elevated temperature with acetic acid in the presence of an esterification catalyst and an entrainer which is sparingly soluble in water and which forms a minimum boiling point azeotrope therewith to form a product comprising entrainer, methyl acetate and water, and in a distillation column, recovering from the product an overhead fraction comprising methyl acetate characterised in that from an intermediate point in the column there is removed a liquid sidestream fraction comprising water and entrainer.

2. A process according to claim 1 characterised in that the methanol and acetic acid are continuously fed to the esterification reaction vessel containing entrainer, acetic acid and esterification catalyst to produce a product comprising entrainer, unreacted methanol, methyl acetate and water, distilling from the product in the distillation column an overhead fraction comprising methyl acetate, removing from the intermediate point in the column a liquid sidestream fraction comprising water, entrainer and methyl acetate, separating methyl acetate and entainer in the sidestream fraction from water and returning separated methyl acetate and entrainer to a point lower in the column than the sidestream fraction removal point.

3. A process according to either claim 1 or claim 2 characterised in that the esterification reaction vessel is a kettle at the base of the distillation column.

4. A process according to any one of the previous claims characterised in that the distillation column contains from 15 to 50 theoretical plates.

5. A process according to any one of the preceding claims characterised in that the elevated temperature is in the range 100 to 120°C.

6. A process according to any one of the preceding claims characterised in that the primary reflux is provided by condensing the overhead fraction in a vertical condenser and returning a portion of the condensate to the column at a reflux ratio in the range 1:2 to 10:1.

7. A process according to any one of the preceding claims characterised in that the sidestream fraction is removed at a point in the column at which the column temperature is between 75 and 90°C.

8. A process according to claim 7 characterised in that the sidestream fraction is removed at a point not less than 10 theoretical plates from the base of the column.

9. A process according to any one of the previous claims characterised in that the column incorporates means for facilitating the removal of a liquid sidestream fraction which means takes the form of a deep weir or chimney tray located immediately below the point in the column from which the sidestream fraction is removed.

10. A process according to any one of the previous claims characterised in that the entrainer is either toluene, diisobutyl ether n-butyl acetate, iso-butyl acetate or methyl ethyl ketone.

11. A process according to claim 10 characterised in that the entrainer is n-butyl acetate.

12. A process according to claim 11 characterised in that the n-butyl acetate is formed 'in-situ' by incorporating n-butanol in the reaction mixture.

13. A process for the production of methyl acetate which process comprises continuously feeding methanol and acetic acid to a reaction kettle surmounted by a distillation column, which kettle contains entrainer, acetic acid and esterification catalyst at elevated temperature, thereby producing a product comprising methyl acetate, water, unreacted methanol, unreacted acetic acid, catalyst and entrainer, removing from the column an overhead fraction comprising principally methyl acetate together with some methanol and water, condensing the overhead fraction, characterised in that the process comprises returning a portion of the condensed overhead fraction to the column as primary reflux, leaving in the reaction kettle a residue fraction comprising catalyst, acetic acid, entrainer and some water, removing from an intermediate point in the column a liquid sidestream fraction comprising water, methyl acetate and entrainer together with some methanol, separating the sidestream fraction by decantation into an upper organic phase comprising principally entrainer and some methyl acetate and a lower aqueous phase comprising principally water and some methyl acetate and methanol and returning the separated organic phase to the column at a point lower in the column than the sidestream fraction removal point.

**Patentansprüche**

1. Verfahren zur Herstellung von Methylacetat durch Umsetzung von Methanol in einem Veresterungs-Reaktionsbehälter bei erhöhter Temperatur mit Essigsäure in Anwesenheit eines Veresterungskatalysators und eines Schleppmittels, welches in Wasser kaum löslich ist und mit ihm ein Minimum-Siedepunkt-Azeotrop bildet, unter Bildung eines Produktes aus Schleppmittel, Methylacetat und Wasser, und Gewinnung einer Überkopffraktion im wesentlichen bestehend aus Methylacetat aus dem Produkt in einer Destillationskolonne, dadurch gekennzeichnet, daß von einem Zwischenpunkt in der Kolonne eine flüssige Nebenstrom-Fraktion aus Wasser und Schleppmittel entfernt wird.

2. Verfahren gemäß Anspruch 1, dadurch gekennzeichnet, daß das Methanol und die Essigsäure kontinuierlich in den Veresterungs-Reaktionsbehälter enthaltend das Schleppmittel, Essigsäure und Veresterungskatalysator eingeführt werden zur Herstellung eines Produktes aus Schleppmittel, nichtumgesetzten Methanol, Methylacetat und Wasser, aus dem Produkt in der Destillationskolonne eine Überkopffraktion aus Methylacetat abdestilliert wird, von dem Zwischenpunkt in der Kolonne eine flüssige Nebenstrom-Fraktion aus Wasser, Schleppmittel und Methylacetat abgezogen wird, Methylacetat und Schleppmittel in der Nebenstrom-Fraktion von dem Wasser abgetrennt werden und abgetrenntes Methylacetat und Schleppmittel zu einem Punkt in der Kolonne unterhalb des Punktes, an dem die Nebenstrom-Fraktion abgezogen wird, rückgeführt werden.

3. Verfahren gemäß Anspruch 1 oder 2, dadurch gekennzeichnet, daß der Veresterungs-Reaktionsbehälter ein Kessel ist an der Basis der Destillationskolonne.

4. Verfahren gemäß einem der vorgehenden Ansprüche, dadurch gekennzeichnet, daß die Destillationskolonne 15 bis 50 theoretische Böden aufweist.

5. Verfahren gemäß einem der vorgehenden Ansprüche, dadurch gekennzeichnet, daß die erhöhte Temperatur im Bereich von 100 bis 120°C liegt.

6. Verfahren gemäß einem der vorgehenden Ansprüche, dadurch gekennzeichnet, daß der primäre Rückfluß geliefert wird durch Kondensation der Überkopffraktion in einem vertikalen Kondensator und Rückführung eines Teils des Kondensats in die Kolonne mit einem Rückflußverhältnis im Bereich von 1:2 bis 10:1.

7. Verfahren gemäß einem der vorgehenden Ansprüche, dadurch gekennzeichnet, daß die Nebenstrom-Fraktion entfernt wird an einem Punkt in der Kolonne bei dem die Kolonnentemperatur zwischen 75 und 90°C liegt.

8. Verfahren gemäß Anspruch 7, dadurch gekennzeichnet, daß die Nebenstrom-Fraktion abgezogen wird an einem Punkt nicht weniger als 10 theoretische Böden entfernt von der Basis der Kolonne.

9. Verfahren gemäß einem der vorgehenden Ansprüche, dadurch gekennzeichnet, daß die Kolonne Mittel aufweist zur Erleichterung des Abziehens einer flüssigen Nebenstrom-Fraktion, wobei die Mittel die Form eines tiefen Überlaufs oder eines Kaminbodens unmittelbar unterhalb des Punktes in der Kolonne von dem die Nebenstrom-Fraktion abgezogen wird annehmen.

10. Verfahren gemäß einem der vorgehenden Ansprüche, dadurch gekennzeichnet, daß das Schleppmittel entweder Toluol, Diisobutylether, n-Butylacetat, iso-Butylacetat oder Methylethylketon ist.

11. Verfahren gemäß Anspruch 10, dadurch gekennzeichnet, daß das Schleppmittel n-Butylacetat ist.

12. Verfahren gemäß Anspruch 11, dadurch gekennzeichnet, daß das n-Butylacetat in situ gebildet wird durch Einverliebung von n-Butanol in die Reaktionsmischung.

13. Verfahren zur Herstellung von Methylacetat wobei Methanol und Essigsäure kontinuierlich in einen Reaktionskessel eingeführt werden, auf den eine Destillationskolonne aufgebaut ist, wobei der Kessel Schleppmittel, Essigsäure und Veresterungskatalysator bei erhöhter Temperatur enthält, wodurch ein Produkt aus Methylacetat, Wasser, nicht-reagiertem Methanol, nichtreagierter Essigsäure, Katalysator und Schleppmittel gebildet wird, aus der Kolonne eine Überkopffraktion im wesentlichen bestehend aus Methylacetat zusammen mit etwas Methanol und Wasser abgezogen wird, die Überkopffraktion kondensiert wird, dadurch gekennzeichnet, daß in dem Verfahren ein Teil der kondensierten Überkopffraktion in die Kolonne als primärer Rücklauf zurückgeführt wird, wobei in dem Reaktionskessel eine Restfraktion hinterbleibt aus Katalysator, Essigsäure, Schleppmittel und etwas Wasser, von einem Zwischenpunkt in der Kolonne eine flüssige Nebenstrom-Fraktion aus Wasser, Methylacetat und Schleppmittel zusammen mit etwas Methanol abgezogen wird, die Nebenstrom-Fraktion durch Dekantierung in eine obere organische Phase in wesentlichen aus Schleppmittel und etwas Methylacetat und eine niedrigere wässrige Phase im wesentlichen aus Wasser und etwas Methylacetat und Methanol getrennt wird und die abgetrennte organische Phase in die Kolonne an einem Punkt unterhalb des Punktes in der Kolonne, an dem die Nebenstrom-Fraktion abgezogen wird zurückgeführt wird.

## Revendications

1. Procédé de production d'acétate de méthyle, ce procédé comprenant la réaction, dans un réacteur d'estérification, du méthanol à température élevée avec l'acide acétique en présence d'un catalyseur d'estérification et d'un entraîneur qui est peu soluble dans l'eau et forme avec elle un azéotrope à point minimal d'ébullition, pour former un produit comprenant l'entraîneur, l'acétate de méthyle et l'eau et, dans une colonne de distillation, la récupération, à partir du produit, d'une fraction de tête comprenant l'acétate de méthyle, procédé caractérisé en ce qu'on enlève d'un point intermédiaire de la colonne une fraction liquide formant courant latéral et comprenant de l'eau et l'entraîneur.

2. Procédé selon la revendication 1, caractérisé en ce qu'on introduit continuellement le méthanol et l'acide acétique dans le réacteur d'estérification contenant l'entraîneur, l'acide acétique et du catalyseur d'estérification, pour obtenir un produit contenant l'entraîneur, du méthanol inaltéré, l'acetate de méthyle et de l'eau, on distille du produit, dans la colonne de distillation, une fraction de tête comprenant l'acétate de méthyle, on enlève du point intermédiaire de la colonne une fraction liquide de courant latéral comprenant de l'eau, l'entraîneur et l'acétate de méthyle, on sépare l'acétate de méthyle et l'entaîneur, présent dans la fraction du courant latéral, d'avec l'eau et l'on recycle l'acétate de méthyle et l'entraîneur, ainsi séparés, vers un point de colonne situé au-dessous du point d'enlèvement de la fraction formant courant latéral.

3. Procédé selon l'une des revendications 1 et 2, caractérisé en ce que le réacteur d'estérification est une chaudière située à la base de la colonne de distillation.

4. Procédé selon l'une quelconque des revendications précédentes, caractérisé en ce que la colonne de distillation contient de 15 à 50 plateaux théoriques.

5. Procédé selon l'une quelconque des revendications précédentes, caractérisé en ce que la température élevée se situe entre 100 et 120°C.

6. Procédé selon l'une quelconque des revendications précédentes, caractérisé en ce qu'on obtient le reflux primaire en condensant la fraction de tête dans un condenseur vertical et en renvoyant vers la colonne une partie du condensat, selon un taux de reflux compris entre 1:2 et 10:1.

7. Procédé selon l'une quelconque des revendications précédentes, caractérisé en ce qu'on retire la fraction formant courant latéral, en un point de la colonne où la température de la colonne se situe entre 75 et 90°C.

8. Procédé selon la revendication 7, caractérisé en ce qu'on retire la fraction formant courant latéral en un point éloigne d'au moins 10 plateaux théoriques de la base de la colonne.

9. Procédé selon l'une quelconque des revendications précédentes, caractérisé en ce que la colonne comporte un moyen pour faciliter l'enlèvement d'une fraction liquide de courant latéral, ce moyen prenant la forme d'un plateau à déversoir profond ou à cheminée, situé immédiatement au-dessous du point de la colonne d'où l'on enlève la fraction formant courant latéral.

10. Procédé selon l'une quelconque des revendications précédentes, caractérisé en ce que l'entraîneur est le toluène, l'éther-oxyde de di-isobutyle, l'acétate de n-butyle, l'acétate d'isobutyle ou la méthyl éthyl cétone.

11. Procédé selon la revendication 10, caractérisé en ce que l'entraîneur est l'acétate de n-butyle.

12. Procédé selon la revendication 11, caractérisé en ce que l'acétate de n-butyle est formé sur place par incorporation de n-butanol au mélange réactionnel.

13. Procédé de production d'acétate de méthyle, ce procédé comprenant l'introduction en continu du méthanol et de l'acide acétique dans une chaudière de réaction surmontée d'une colonne de distillation, cette chaudière contenant de l'entraîneur, de l'acide acétique et de catalyseur d'estérification à température élevée, ce qui donne un produit comprenant de l'acétate de méthyle, de l'eau, du méthanol inaltéré, de l'acide acétique inaltéré, du catalyseur et de l'entraîneur, l'enlèvement de la colonne d'une fraction de tête comprenant surtout de l'acétate de méthyle ainsi qu'un peu de méthanol et d'eau, la condensation de la fraction de tête, procédé caractérisé en ce qu'il comprend le recyclage, à titre de reflux primaire, d'une partie de la fraction de tête condensée vers la colonne, en laissant dans la chaudière de réaction une fraction formant résidu et comprenant du catalyseur, de l'acide acétique, de l'entraîneur et un peu d'eau, l'enlèvement, d'un point intermédiaire de la colonne, d'une fraction de liquide formant courant latéral et comprenant de l'eau de l'acétate de méthyle et de l'entraîneur avec un peu de méthanol, la séparation de la fraction formant courant latéral, par décantation, en une phase organique supérieure comprenant principalement l'entraîneur et un peu d'acétate de méthyle et en une phase aqueuse inférieure comprenant principalement l'eau et un peu d'acétate de méthyle et de méthanol, et le recyclage vers la colonne, en un point de la colonne situé au-dessous du point d'enlèvement de la fraction formant courant latéral, de la phase organique séparée.